# EUROPEAN PATENT APPLICATION

(11) **EP 3 804 655 A1**
(43) Date of publication of application: **14.04.2021**
(21) Application number: 20200708.4
(22) Date of filing: 08.10.2020
(51) Int. Cl.: A61C 7/08, A61F 5/56, A61C 7/36

(54) **BALANCING DEVICE FOR THE TEMPOROMANDIBULAR JOINT**

(30) Priority: 10.10.2019 IT 201900018518
(71) Applicant: FKS Holding Srl, 47521 Cesena FC (IT)
(72) Inventor: SINTUCCI, Fabrizio, 47521 Cesena (IT)
(74) Representative: Busca, Andrea

(57) **Abstract**

The present invention relates to a biofunctional balancing device (1) for the temporomandibular joint where the device is a bite guard with at least one support surface (6, 7) for supporting the cusps of the teeth of a dental arch inclined in two specular zones to form two opening ramps (10) of the canines and/or molars.

## Description

The present invention relates to a biofunctional balancing device for the temporomandibular joint.

### STATE OF THE ART

The orthodontic sector, and in particular gnathology, deals in general with the craniofacial morphostructure, and in particular the aesthetic and functional rehabilitation of the dental arches with respect for the temporomandibular joints, the gum tissues and the chewing, speech and swallowing functions.

Malformations in general are treated through the application of corrective devices.

Although fixed and mobile devices exist in the sector, they are generally annoying to wear and therefore invasive, as they concentrate on the individual part of the mouth to be treated and neglect the interaction with other parts. A typical example is devices for enlarging the palate, which do not consider the posture of the tongue and interaction with other soft tissues.

A typical example is devices for enlarging the palate which, in general remain hanging by pressure between the opposite walls of the dental arches. Their effect is continuous which is why they require metal adjustment mechanisms for restoring pressure as the palate is enlarged. These mechanisms are delicate and must be left partially for users to handle as their adjustment is very frequent, thus being difficult to manage, especially for children. Finally, such mechanisms inevitably press directly on the mouth's soft tissues, being annoying.

There are also devices that act by exploiting the biting action between the maxilla and the mandible. They do not require adjustment through mechanisms as they do not hang from the joint, but are clamped between opposing teeth. Their action is therefore "fluctuating" as it depends on the clamping intensity of the bite, which during the day varies continuously and unconsciously.

As biting is a natural action, these devices are not invasive, and can also be worn by children without any annoyance. They also stimulate the chewing impulse, thus increasing their efficacy.

These devices are biofunctional balancing devices for the temporomandibular joint, commonly known as "bite guards", and in general they are used to solve occlusion problems, i.e. alignment of the teeth between the upper and lower arches.

In general they do not interact with the soft tissues of the mouth.

Although they can generate some effects in enlarging the palate, the need remains latent in the sector to increase their efficacy in that sense through a specific effect.

It is an object of the present invention to overcome all, or some, of the drawbacks of the prior art.

In particular, a preferred object of the present invention is that of providing a specific biofunctional balancing device for the temporomandibular joint for the enlargement or narrowing of the palate or the maxilla by exploiting the biting action between the mandible and the maxilla.

A further object of the present invention is that of providing a biofunctional balancing device for the temporomandibular joint that is effective and comfortable to wear.

In particular, it is a preferred object of the present invention to provide a device for the enlargement of the palate that when worn also enables the tongue to be placed in biofunctional balance, generating a synergistic effect.

A further object of the present invention is that of respecting the soft tissues of the mouth.

### GENERAL INTRODUCTION

According to a first general aspect, the present invention relates to a balancing device for the temporomandibular joint where the device is a bite guard with at least one resting surface for the cusps of the teeth of a dental arch, where the resting surface is inclined in two specular areas forming two opening or nearing ramps for the canines and/or molars.

A bite guard means in general a single block of elastic material, substantially U-shaped, for being clamped between the teeth with a biting action.

Opening means the distancing of specular teeth from one another. Nearing is the opposite operation, i.e. the nearing of specular teeth.

Preferably, the device is a bite guard comprising an occlusal plane wall (5), U-shaped, intended to be interposed between the two dental arches of a user, the U-shape comprises two arms facing one another, intended to be interposed between the molars and the canines of the two dental arches, and a front joining zone of the two arms, intended to be interposed between the incisors, where the two arms each have an upper surface and a lower surface opposite one another intended to rest on the cusps of the teeth of the mandible and the maxilla, respectively, which they face, characterized in that at least one of said surfaces of at least one of said arms is at least partially inclined towards the inside or outside of the U-shape so as to be intended to exercise on said cusps a corresponding thrust towards said inside and/or outside during the biting action. The inclined surfaces form said ramps.

According to some preferred embodiments of the invention said inclination is greater than that between the highest and lowest cusp of at least one molar, so as to have a prevalent thrust on the higher cusp.

Preferably both arms have at least one of said surfaces at least partially inclined, where said surfaces are oriented on the same side of the occlusal plane so as to be intended to face the same dental arch, and have a specular inclination to one another so that both are intended to exercise on the respective cusps a thrust towards the inside or outside of the U-shape.

Preferably, said inclination is in one point intended to face at least one canine.

Preferably, the ramps are on an upper resting surface of the bite guard.

According to some preferred embodiments the device is made as a single block of elastic material,
the device comprises a base wall (5) which extends in a main plane (P) coinciding with the occlusal plane,
the base wall (5) is U-shaped and has dimensions such as to be interposed between the dental arches of the temporomandibular joint to be clamped between one another,
the wall (5) has respective upper and lower surfaces for contact with the cusps of the teeth,
characterized in that at least one of the two upper and lower surfaces is inclined with respect to the extension plane (P) at least in two specular zones extended between one another between the canines and the molars, the inclination being such for which the surface forms two ramps for the sliding of the teeth from the inside towards the outside of the mouth, thus generating a symmetrical distancing effect between the teeth in the two zones.

In that case, the inclined surface is preferably the upper surface.

According to a general preferable characteristic, the single block comprises an upper distal wall projecting upwards from the base wall and intended to be interposed between the upper dental arch and the corresponding lip, the upper distal wall preferably extends along the whole outer perimeter of the base wall.

It is also possible that the single block comprises a lower distal wall projecting downwards from the base wall and intended to be interposed between the lower dental arch and the corresponding lip, the lower distal wall preferably extends along the whole outer perimeter of the base wall.

In general, the single block comprises a lingual ramp intended to rest below the tongue and inclined upwards proceeding from the inside to the outside of the mouth so as to guide the tongue towards the palatine spot.

In that case the lingual ramp preferably comprises an area of discontinuity to create a stimulus for the tongue.

More preferably said area of discontinuity is in a plane of symmetry of the two specular inclined zones.

Even more preferably said area of discontinuity comprises a ball or the like.

### DETAILED DESCRIPTION

Further characteristics and advantages of the present invention will become clearer from the following detailed description of preferred embodiments thereof, with reference to the appended drawings and provided by way of an indicative and non-limiting example. In such drawings:
- figure 1 schematically shows a preferred embodiment of a device according to the present invention in a perspective view;
- figure 2 shows the device of figure 1 in a view from above;
- figure 3 shows the device of figure 1 in a view from below;
- figure 4 shows the device of figure 1 in a section according to the plane IV of figure 2;

With reference to the figures, a biofunctional balancing device for the temporomandibular joint is illustrated, indicated overall by reference number 1.

The device 1 is a bite guard. A practical embodiment envisages a single block of elastic polymer material, e.g. obtained by die casting.

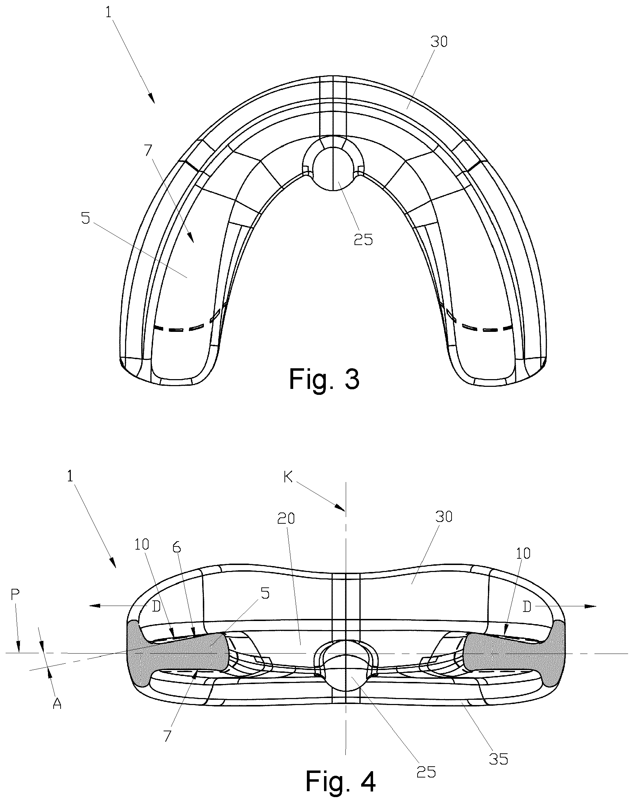

In general, the device 1 comprises a base wall 5 that extends in a main plane P coinciding with the occlusal plane (figure 4), also therefore known as the occlusal plane wall. The base wall 5 is U-shaped and has dimensions such as to be interposed between the dental arches of the temporomandibular joint to be clamped between one another.

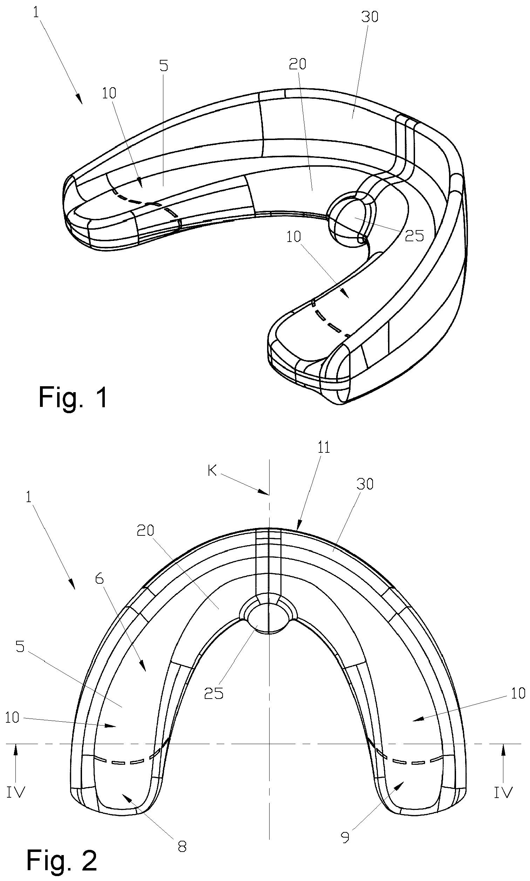

As can be seen more clearly in figure 2, the U-shape has two rear arms 8 and 9, facing with respect to a bisection plane K of the U-shape orthogonal to the main plane P. The arms are joined to one another by a front portion 11.

Each arm 8, 9 has respective upper 6 and lower 7 surface for contact with the cusps of the teeth, where at least one of the two upper and lower surfaces is at least partially inclined towards the inside or the outside of the U-shape, i.e. it forms non-orthogonal angles both with the base plane P and with the bisection plane K. In this way, said inclination forms a support and sliding ramp 10 for the cusps of the teeth towards the bisection plane K or away from it. Preferably the occlusal wall comprises at least a pair of ramps, comprising a ramp on each arm facing the same side of the occlusal wall, so as to be preferably specular to one another with respect to the bisection plane K. Each specular ramp 10 is present at least in part of a zone corresponding to the molars and preferably also to the canines. For example, each ramp has an extension in the occlusal plane such as to match with respective groups of specular teeth, e.g. the preferred group comprises adjacent molars and canines, however it is not excluded that the ramps extend so as to match with entire semi-arches, i.e. from the incisors to the molars, so as to be joined to one another with continuity. Also, it is not excluded that they extend to match only with one tooth or with groups of teeth of the same type, e.g. with molars or one or more of them.

The specular ramps 10 are preferably facing above the bite guard, i.e. realized from the upper surface 6, so as to interact with the upper dental arch and cause the enlargement of the palate. An opposite inclination to move the teeth closer is not excluded, just as it is not excluded that additionally or alternatively to the ramps 10 on the upper surface 6 there is at least one ramp 10, preferably a pair of ramps 10, such as those described, on the lower surface 7.

The inclination A of each ramp 10 with respect to the main plane P is preferably such for which the ramp comes into contact with the highest cusp of at least one molar (vestibular cusps), or of a group of molars. This does not exclude that this may be a first contact and that during the deformation of the bite guard device due to the bite the ramp 10 may come into contact also with the lowest cusp (lingual cusps).

In the embodiment illustrated the arms 8 and 9 define a pair of specular ramps 10 on the upper surface 6 such as to create a distancing effect and therefore an opening effect between the two specular groups of molars and/or canines of the upper dental arch, as can be seen in particular in the section of figure 4 and indicated by the arrows D.

The bite further comprises a ramp for the support of the tongue 20 projecting from the base wall 5 clamped between the dental arches. The tongue support ramp is in turn U-shaped and inclined to rise from the base of the tongue towards the upper dental arch so as to guide the tongue towards the palatine spot.

At the base of the tongue support ramp 20 there may optionally be a discontinuity 25, such as for example a ball or the like, to stimulate the tongue.

The bite guard is completed by a U-shaped front wall 30, preferably extending along the entire outer perimeter of the base wall 5 and projecting upwards to be interposed between the upper dental arch and the relative lip.

A corresponding lower front wall is indicated by number 35.

It is also possible to realize one or more rear walls projecting upwards from the base wall 5, for being interposed between the dental arches and the inside of the mouth, so that the dental arches come into a housing channel. It is observed that the examples illustrated are free from such rear walls and are therefore free from functional parts that exercise a thrust on the inner side surfaces of the dental arches.

### GENERAL INTERPRETATION OF TERMS

In understanding the object of the present invention, the term "comprising" and its derivatives, as used herein, are intended as open-ended terms that specify the presence of declared characteristics, elements, components, groups, integers and/or steps, but do not exclude the presence of other undeclared characteristics, elements, components, groups, integers and/or steps. The above also applies to words, which have similar meanings, such as the terms "comprised", "have" and their derivatives. Furthermore, the terms "part", "section", "portion", "member" or "element" when used in the singular can have the double meaning of a single part or a plurality of parts. As used herein to describe the above executive embodiment(s), the following directional terms "forward", "backward", "above", "under", "vertical", "horizontal", "below" and "transverse", as well as any other similar directional term, refers to the embodiment described in the operating position. Finally, terms of degree, such as "substantially", "about" and "approximately", as used herein, are intended as a reasonable amount of deviation of the modified term such that the final result is not significantly changed.

While only selected embodiments have been chosen to illustrate the present invention, it will be apparent from this description to those skilled in the art that various modifications and variations can be made without departing from the scope of the invention as defined in the appended claims. For example, the size, shape, position or orientation of the various components can be modified as needed and/or desired. The components shown directly connected or in contact with each other can have intermediate structures arranged between them. The functions of one element can be performed by two and vice versa. The structures and functions of one embodiment can be adopted in another embodiment. All the advantages of a particular embodiment do not necessarily have to be present at the same time. Any characteristic that is original compared to the prior art, alone or in combination with other characteristics, should also be considered a separate description of further inventions by the applicant, including the structural and/or functional concepts embodied by such characteristics. Therefore, the foregoing descriptions of the embodiments according to the present invention are provided for illustrative purposes only and not for the purpose of limiting the invention as defined by the appended claims and the equivalents thereof.

## Claims

1. A balancing device for the temporomandibular joint where the device is a bite guard comprising an occlusal plane wall (5), U-shaped, intended to be interposed between the two dental arches of a user, the U-shape comprises two arms facing one another, intended to be interposed between the molars and the canines of the two dental arches, and a front joining zone of the two arms, intended to be interposed between the incisors, where the two arms each have an upper surface and a lower surface opposite one another intended to rest on the cusps of the teeth of the mandible and the maxilla, respectively, which they face, **characterized in that** at least one of said surfaces of at least one of said arms is at least partially inclined towards the inside or outside of the U-shape so as to be intended to exercise on said cusps a corresponding thrust towards said inside and/or outside during the biting action.

2. Device according to claim 1, **characterized in that** said inclination is greater than that between the highest and lowest cusp of at least one molar, so as to have a prevalent thrust on the higher cusp.

3. Device according to any one of the preceding claims, **characterized in that** both arms have at least one of said surfaces at least partially inclined, where said surfaces are oriented on the same side of the occlusal plane so as to be intended to face the same dental arch, and have a specular inclination to one another so that both are intended to exercise on the respective cusps a thrust towards the inside or outside of the U-shape.

4. Device according to any one of the preceding claims, **characterized in that** said inclination is in a point intended to be facing at least one canine.

5. Device according to any one of the preceding claims, **characterized in that** said inclination is on an upper support surface (6) of the bite guard (1).

6. Device according to any one of the preceding claims, **characterized in that** it comprises an upper distal wall (30) projecting upwards from the occlusal plane wall (5) and intended to be interposed between the upper dental arch and the corresponding lip, the upper distal wall (30) preferably extends for the entire outer perimeter of the base wall (5).

7. Device according to any one of the preceding claims, **characterized in that** it comprises a lower distal wall (35) projecting downwards from the occlusal plane wall (5) and intended to be interposed between the lower dental arch and the corresponding lip, the lower distal wall (35) preferably extends for the entire outer perimeter of the base wall (5).

8. Device according to any one of the preceding claims, **characterized in that** it comprises a lingual ramp (20) intended to rest below the tongue and inclined upwards proceeding from the inside to the outside of the mouth so as to guide the tongue towards the palatine spot.

9. Device according to claim 8, **characterized in that** the lingual ramp (20) comprises a discontinuity (25) for creating a stimulus for the tongue.

10. Device according to claim 9, **characterized in that** said discontinuity zone (25) is in a bisection plane of the U-shape (K) orthogonal to the occlusal plane.

11. Device according to claim 9, **characterized in that** said discontinuity zone comprises a ball (25) or the like.

12. Device according to any one of the previous claims, **characterized in that** it is a single block of elastic material.
